# EUROPEAN PATENT SPECIFICATION

(11) **EP 1 785 158 B1**
(45) Date of publication and mention of the grant of the patent: **17.06.2009**
(21) Application number: 05024531.5
(22) Date of filing: 10.11.2005
(51) Int. Cl.: A61M 25/06, A61M 5/32

(54) **Indwelling needle**
Verweilnadel
Aiguille à demeure

(43) Date of publication of application: 16.05.2007
(73) Proprietor: Medikit Co., Ltd., Tokyo (JP)
(72) Inventor: Nakajima, Hiroaki, Bunkyo-ku, Tokyo (JP)
(74) Representative: Kreutzer, Ulrich

(56) References cited:
- EP-A- 0 763 369
- GB-A- 2 375 053

## Description

### BACKGROUND OF THE INVENTION

### FIELD OF THE INVENTION

The present invention relates to an indwelling needle for an infusion set, which has a down-sized constitution and is capable of regulating the amount of a blood back-flow.

### DESCRIPTION OF THE RELATED ART

Indwelling needles are used to infuse various medicinal fluids into vascular systems. In some case, an indwelling needle is provided with an inner needle for being stuck into a vascular system and an outer needle coaxially fit on the inner needle. After sticking, the inner needle may be extracted but the outer needle is left inserted and used for infusion.

Japanese Patent Application Laid-open JP2002-102347 discloses an art of an indwelling needle assembly provided with a needle cover and blood monitoring means. The needle cover is configured to cover the inner needle and the blood monitoring means and hence assures safe disposal thereof without accidental sticking after use. The bloodmonitoringmeans is movably housed in the needle cover and provided with a chamber for receiving back-flow of blood. When the inner needle is inserted in the vascular system, an operator can observe the back-flow of the blood pooled in the chamber from the exterior so as to verify the inner needle is regularly inserted in the vascular system.

Another state of art indwelling needle is known from the disclosure made in EP-A-0 763 369.

### SUMMARY OF THE INVENTION

The chamber of the blood monitoring means needs to keep receiving the back-flow of the blood over a considerable period of time and is hence necessary to have a considerable capacity. Therefore the blood monitoring means necessarily has considerable dimensions. Further the cover needs to have enough dimensions to cover such blood monitoring means. It gives rise to a problem that increase in size of the indwelling needle is inevitable. Such increase in size results in operator's disadvantage in view of easiness of delicate handling and operation in practical use.

The present invention has been carried out in view of the above problem and is intended for providing an indwelling needle for an infusion set, which has a down-sized constitution and is capable of regulating the amount of a blood back-flow.

According to an aspect of the present invention as claimed, an indwelling needle is provided with an inner needle hub; an inner needle fixedly supported by the inner needle hub; a cover configured to cover the inner needle after use; and a flexible blood monitor being absorptive to blood, an end of which is linked with the inner needle hub.

Preferably, the blood monitor is configured to regulate quickness of spread of the blood. More specifically, the indwelling needle is further provided with a plug configured to fix the blood monitor with the inner needle hub, which is capable of regulate tightness of fixation of the blood monitor with the inner needle hub so as to regulate quickness of spread of the blood.

More preferably, the blood monitor is formed to be of a long and thin string shape.

Further preferably, the cover is capable of expanding. More specifically, the cover is composed of an outer cylinder and an inner cylinder. The inner cylinder is movably housed in the outer cylinder and detachably supports the inner needle hub. Still preferably, the indwelling needle is further provided with an elastic member configured to keep repulsive force to separate the inner cylinder and the inner needle hub. Still preferably, the indwelling needle is further provided with a latch piece configured to lock the inner cylinder and the inner needle hub in an immovable state relative to the outer cylinder.

### BRIEF DESCRIPTION OF THE DRAWINGS

Fig. 1 is a schematic drawing schematically illustrating a state before use of an indwelling needle in accordance with an embodiment of the present invention;
Fig. 2 is a schematic drawing schematically illustrating a state subject for disposal after use of the indwelling needle;
Fig. 3 is a schematic drawing schematically illustrating a state before use of an inner needle and a blood monitor; and
Fig. 4 is a schematic drawing schematically illustrating a state subject for disposal after use of the inner needle and the blood monitor.

### DETAILED DESCRIPTION OF THE PREFERRED EMBODIMENT

Throughout the specification, claims and the drawings, distal ends of constituent members are defined as ends directed to where a needlepoint points and proximal ends are defined as ends opposed thereto.

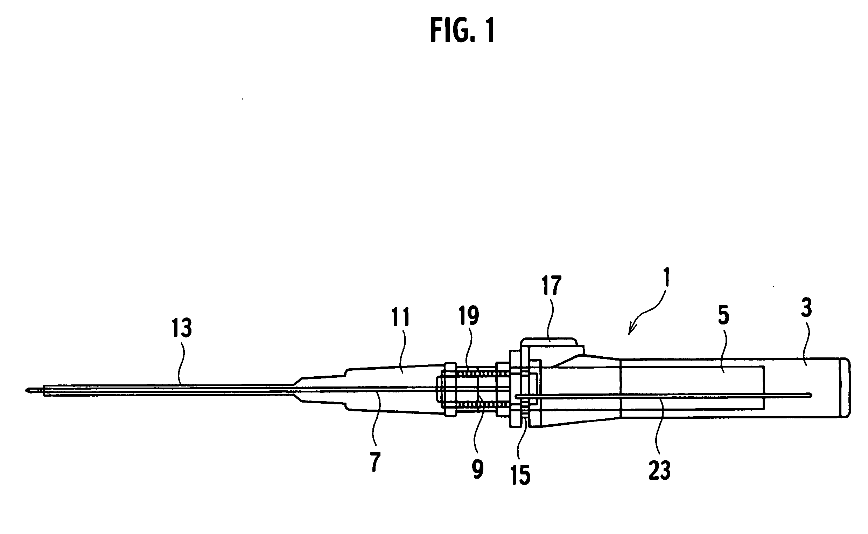

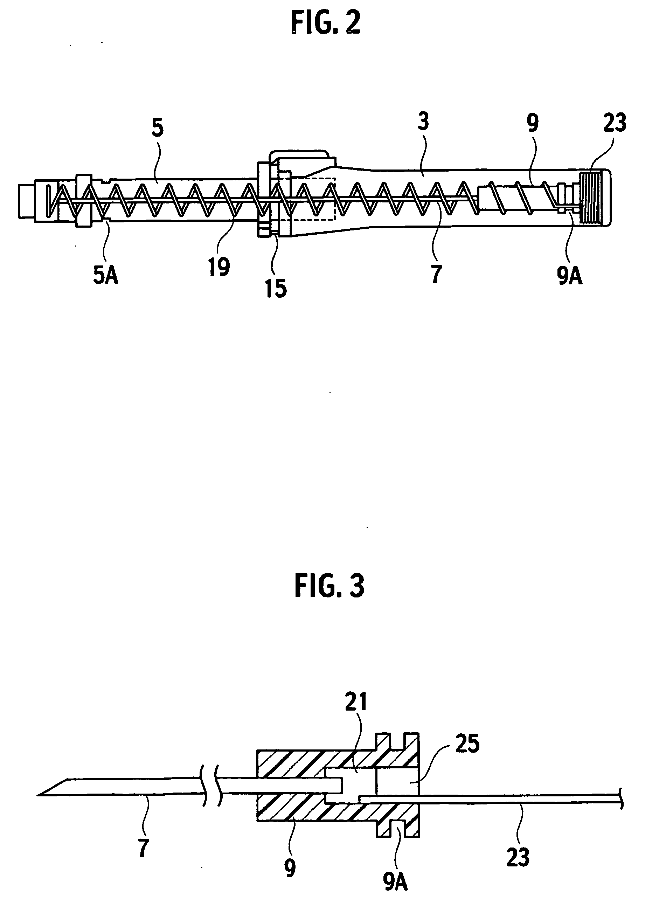

Referring to Fig. 1, an indwelling needle 1 in accordance with an embodiment of the present invention is provided with a transparent outer cylinder 3 having an opened distal end and a closed proximal end. The outer cylinder 3 is made of polycarbonate or any other transparent synthetic resin to allow visual observation of the interior. An inner cylinder 5 is housed in the outer cylinder 3 and axially movable relative to the outer cylinder 3 so as to project therefrom as shown in Fig. 2. A distal end of the inner cylinder 5 detachably supports an inner needle hub 9. An inner needle 7, which is formed to be sharp-pointed for being stuck into a patient body, is fixedly supported by the inner needle hub 9. An outer needle hub 11 is detachably fit on a distal end of the inner needle hub 9 and supports a proximal end of an outer needle 13. The outer needle 13 is formed to be tubular and the inner needle 7 is inserted and slidably fit therein.

The outer cylinder 3 is provided with an operation member 17 near the distal end thereof. The operation member 17 is provided with an annular latch piece 15, which is movable in a direction perpendicular to the axis of the outer cylinder 3. The latch piece 15 is configured to lock the inner cylinder 5 and the inner needle hub 9 so as to be in an immovable state before use of the indwelling needle 1.

More specifically, a lower part of the inner cylinder 5 is partly cut out to form a latching recess portion 5A as shown in Fig. 2 and the inner needle hub 9 is provided with a latching recess portion 9A in the vicinity of the proximal end thereof. The latching recess portion 5A and the latching recess portion 9A are so dimensioned as to be aligned in the axial direction in a state before use as shown in Fig. 1. The latch piece 15 latches with the latching recess portion 5A and further goes through the latching recess portion 5A to latch with the latching recess portion 9A in the state before use, thereby the latch piece 15 locks the inner cylinder 5 and the inner needle hub 9 so as to be in the immovable state before use.

An elastic member 19 such as a coil spring is installed in the inner cylinder 5 to span an inner surface of the distal end of the inner cylinder 5 and the inner needle hub 9. In the state before use, the elastic member 19 is compressed as shown in Fig. 1 and hence keeps repulsive force to separate the inner cylinder 5 and the inner needle hub 9, though the latch piece 15 locks the inner cylinder 5 and the inner needle hub 9. In a case where the operation member 17 is pressed, the latch piece 15 is released from the latching recess portion 5Aand the latching recess portion 9A, then the elastic member 19 expands by means of the repulsive force so that the inner cylinder 5 projects out of the outer cylinder 3 and the inner needle hub 9 recedes toward the proximal end of the outer cylinder 3.

Then the projecting inner cylinder 5 covers a distal portion of the inner needle 7 and the outer cylinder 3 covers a proximal portion of the inner needle 7 as shown in Fig. 2. More specifically, the inner cylinder 5 and the outer cylinder 3 cooperatively function as a cover capable of expanding and contracting and configured to cover the inner needle 7 after use.

Referring to Fig. 3, the inner needle hub 9 is provided with a small chamber 21 having an opening at the proximal end thereof. An end of a blood monitor 23, which is made to be of a long and thin string shape, of a line or of a fiber, is inserted into the chamber 21 and fixed by a plug 25 for closing the opening of the chamber 21. The opening of the chamber 21 may be tapered so that tightness of the fixation of the blood monitor 23 with respect to the opening can be regulated by means of regulation of insertion length of the plug 25.

The blood monitor 23 is made to be absorptive and flexible. Although any material having absorptive capacity and flexibility may be applied thereto, the blood monitor 23 is preferably made of any absorptive and flexible material such as felt, cloth or paper of pulp, cotton or any absorptive resin. More preferably thebloodmonitor 23 has a core made of such a material and flexible laminates thereon. Thereby the blood monitor 23 allows back-flow of blood to spread out of the chamber 21 when the indwelling needle 1 is inserted into a patient body. Quickness of spread of the blood may be regulated in advance by means of the tightness of the fixation of the blood monitor 23 by the plug 25.

In accordance with the above described constitution, in a case where the inner cylinder 5 and the inner needle hub 9 are fixed by means of the latch piece 15 as shown in Fig. 1, the blood monitor 23 is in a state of linearly expanding as shown in Fig. 3. When the needle is inserted into a vascular system of a patient, back-flow of blood flows into the chamber 21 of the inner needle hub 9.

Then the blood spreads through the blood monitor 23 to the exterior. Therefore regularity of insertion of the needle into the vascular system can be verified by means of observation of the back-flow of the blood and the quickness thereof. After verifying the regularity of the needle insertion into the vascular system, the operation member 17 is operated so as to cancel fixation of the inner cylinder 5 and the inner needle hub 9. Then the repulsive force of the elastic member 19 pulls the inner needle 7 out of the outer needle 13 and pushes the inner cylinder 5 to project from the outer cylinder 3.

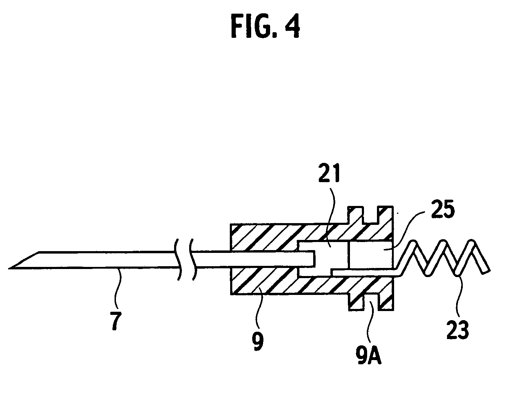

As the inner needle 7 goes out of the outer needle 13 and the inner cylinder 5 projects from the outer cylinder 3, the blood monitor 23 is bent and folded between the proximal end of the outer cylinder 3 and the inner needle hub 9 as shown in Figs. 2 and 4. When the inner cylinder 5 completely projects, the distal end of the inner cylinder 5 reaches ahead of the distal end of the inner needle 7. Thereby the distal end of the inner needle 7 is covered with the inner cylinder 5 so as to assure safety. It can be safely subj ect to disposal without accidental sticking. The total length of the outer cylinder 3 and the inner cylinder 5 projecting therefrom is not required to be so greater than the length of the inner needle 7. Therefore the total length at a time of disposal can be made relatively short.

Regularity of insertion of the needle into the vascular system can be verified by means of observation of spread of the blood through the string-like blood monitor 23, thereby there is no need to give considerable dimensions to the chamber 21. The chamber 21 can be small-sized as long as the dimensions of the chamber 21 are enough to connect the proximal end of the inner needle 7 with the blood monitor 23. Therefore the inner needle hub 9 may be made smaller in diameter and shorter in length, thereby the indwelling needle 1 may be made thinner and shorter. It leads to facility in handling and sticking of the indwelling needle 1.

Moreover, in accordance with the aforementioned constitution, because quickness of spread of the blood can be regulated by means of the tightness of the fixation of the blood monitor 23, loss of blood of the patient may be suppressed.

Although the invention has been described above by reference to a certain embodiment of the invention, the invention is not limited to the embodiments described above. Modifications and variations of the embodiment described above will occur to those skilled in the art, in light of the above teachings.

## Claims

1. An indwelling needle (1) comprising:
an inner needle hub (9);
an inner needle (7) fixedly supported by the inner needle hub (9) ;
a cover (3, 5) configured to cover the inner needle (7) after use; and
a flexible blood monitor (23) being absorptive to blood, an end of the blood monitor (23) being linked with the inner needle hub (9).

2. The indwelling needle (1) of claim 1, wherein the blood monitor (23) is configured to regulate quickness of spread of the blood.

3. The indwelling needle (1) of claim 1, further comprising a plug (25) configured to fix the blood monitor (23) with the inner needle hub (9) , the plug (25) being capable of regulate tightness of fixation of the blood monitor (23) with the inner needle hub (9) so as to regulate quickness of spread of the blood.

4. The indwelling needle (1) of any one of claims 1 to 3, wherein the blood monitor (23) is formed to be of a long and thin string shape.

5. The indwelling needle (1) of any one of claims 1 to 4, wherein the cover (3, 5) is capable of expanding.

6. The indwelling needle (1) of any one of claims 1 to 5, wherein the cover (3, 5) comprises an outer cylinder (3) and an inner cylinder (5), the inner cylinder (5) being axially movably housed in the outer cylinder (3) and detachably supporting the inner needle hub (9) so that the inner cylinder (5) is capable of projecting out of the outer cylinder (3) and the inner needle (7) is capable of receding into the outer cylinder (3).

7. The indwelling needle (1) of claim 6, further comprising an elastic member (19) configured to keep repulsive force to separate the inner cylinder (5) and the inner needle hub (9).

8. The indwelling needle (1) of claim 7, further comprising a latch piece (15) configured to lock the inner cylinder (5) and the inner needle hub (9) in an immovable state relative to the outer cylinder (3).

## Patentansprüche

1. Verweilkanüle (1) mit:
einem inneren Kanülenstempel (9);
einer fest von dem inneren Kanülenstempel (9) getragenen, inneren Kanüle (7);
einer Abdeckung (3, 5), die zur Abdeckung der inneren Kanüle (7) nach dem Gebrauch konfiguriert ist; und
einer flexiblen Blutüberwachungsvorrichtung (23), die Blut absorbieren kann, wobei ein Ende der Blutüberwachungsvorrichtung (23) mit dem inneren Kanülenstempel (9) verbunden ist.

2. Verweilkanüle (1) nach Anspruch 1, wobei die Blutüberwachungsvorrichtung (23) zum Regulieren der Schnelligkeit der Blutausbreitung konfiguriert ist.

3. Verweilkanüle (1) nach Anspruch 1, außerdem mit einem Stopfen (25) mit einer Konfiguration zum Befestigen der Blutüberwachungsvorrichtung (23) an dem inneren Kanülenstempel (9), wobei der Stopfen (25) die Dichtigkeit der Befestigung der Blutüberwachungsvorrichtung (23) an dem inneren Kanülenstempel (9) regulieren kann, um dadurch die Schnelligkeit der Blutausbreitung zu regulieren.

4. Verweilkanüle (1) nach einem der Ansprüche 1 bis 3, wobei die Blutüberwachungsvorrichtung (23) zu einer langen und dünnen Schnurform gestaltet ist.

5. Verweilkanüle (1) nach einem der Ansprüche 1 bis 4, wobei sich die Abdeckung (3, 5) ausdehnen kann.

6. Verweilkanüle (1) nach einem der Ansprüche 1 bis 5, wobei die Abdeckung (3, 5) einen äußeren Zylinder (3) und einen inneren Zylinder (5) umfaßt, wobei der innere Zylinder (5) in Axialrichtung bewegbar in dem äußeren Zylinder (3) untergebracht ist und lösbar den inneren Kanülenstempel (9) so trägt, daß der innere Zylinder (5) aus dem äußeren Zylinder (3) hervorstehen kann und die innere Kanüle (7) in den äußeren Zylinder (3) zurückgezogen werden kann.

7. Verweilkanüle (1) nach Anspruch 6, ferner ein elastisches Glied (19) umfassend, das zum Aufrechterhalten einer Abstoßkraft, um den inneren Zylinder (5) und den inneren Kanülenstempel (9) zu trennen, konfiguriert ist.

8. Verweilkanüle (1) nach Anspruch 7, ferner ein Einraststück (15) umfassend, das zum Verriegeln des inneren Zylinders (5) und des inneren Kanülenstempels (9) in unbewegbarem Zustand in bezug auf den äußeren Zylinder (3) konfiguriert ist.

## Revendications

1. Aiguille à demeure (1) comportant :
un embout d'aiguille intérieure (9) ;
une aiguille intérieure (7) supportée de façon fixe par l'embout d'aiguille intérieure (9) ; un capuchon (3, 5) configuré pour recouvrir l'aiguille intérieure (7) après utilisation ; et
un contrôleur de sang flexible (23) qui peut absorber du sang, une extrémité du contrôleur de sang (23) étant liée à l'embout d'aiguille intérieure (9).

2. Aiguille à demeure (1) selon la revendication 1, dans laquelle le contrôleur de sang (23) est configuré pour réguler la rapidité de propagation du sang.

3. Aiguille à demeure (1) selon la revendication 1, comportant en outre un bouchon (25) configuré pour fixer le contrôleur de sang (23) avec l'embout d'aiguille intérieure (9), le bouchon (25) étant capable de réguler la force de la fixation du contrôleur de sang (23) avec l'embout d'aiguille intérieure (9) de façon à réguler la rapidité de propagation du sang.

4. Aiguille à demeure (1) selon l'une quelconque des revendications 1 à 3, dans laquelle le contrôleur de sang (23) est en forme de cordon long et fin.

5. Aiguille à demeure (1) selon l'une quelconque des revendications 1 à 4, dans laquelle le capuchon (3, 5) est capable de se dilater.

6. Aiguille à demeure (1) selon l'une quelconque des revendications 1 à 5, dans laquelle le capuchon (3, 5) comporte un cylindre extérieur (3) et un cylindre intérieur (5), le cylindre intérieur (5) étant logé de façon axialement mobile dans le cylindre extérieur (3) et supporte de manière détachable l'embout d'aiguille intérieure (9) de telle sorte que le cylindre intérieur (5) peut dépasser hors du cylindre extérieur (3) et l'aiguille intérieure (7) se rétracter dans le cylindre extérieur (3).

7. Aiguille à demeure (1) selon la revendication 6, comportant en outre un élément élastique (19) configuré pour maintenir une force répulsive afin de séparer le cylindre intérieur (5) et l'embout d'aiguille intérieure (9).

8. Aiguille à demeure (1) selon la revendication 7, comportant en outre une pièce de verrouillage (15) configurée pour bloquer le cylindre intérieur (5) et l'embout d'aiguille intérieure (9) dans un état immobile par rapport au cylindre extérieur (3).
